(19) Europäisches Patentamt
European Patent Office
Office européen des brevets

(11) EP 0 949 509 A1

(12) EUROPEAN PATENT APPLICATION

(43) Date of publication:
13.10.1999 Bulletin 1999/41

(51) Int. Cl.[6]: G01N 33/577, G01N 33/74

(21) Application number: 99302377.9

(22) Date of filing: 26.03.1999

(84) Designated Contracting States:
AT BE CH CY DE DK ES FI FR GB GR IE IT LI LU MC NL PT SE
Designated Extension States:
AL LT LV MK RO SI

(30) Priority: 30.03.1998 JP 8433398

(71) Applicant:
MITSUI CHEMICALS, INC.
Tokyo (JP)

(72) Inventors:
- Hashimoto, Yoshihide
Funabashi-shi, Chiba (JP)
- Kono, Naoko
Mobara-shi, Chiba (JP)
- Makino, Tadashi
Togane-shi, Chiba (JP)
- Irie, Minoru
Tokyo (JP)

(74) Representative:
Harvey, David Gareth et al
Graham Watt & Co.
Riverhead
Sevenoaks Kent TN13 2BN (GB)

# (54) A method of measurement of human growth hormone

(57) A blood concentration of hGH that has been administered to a subject is measured by a method on the basis of a ratio between blood or urine concentrations of a human growth hormone having a molecular weight of about 22,000 (22K hGH) and a human growth hormone having a molecular weight of about (20K hGH), and a method of diagnosing whether or not hGH has been administered, is performed judged on the basis of a ratio between blood or urine concentrations of 22K hGH and 20K hGH.

The method is applicable to the testing of sports competitors for the use of banned, performance-enhancing hGH substances.

EP 0 949 509 A1

## Description

The present invention relates to a method of determining concentration of an externally-administered human growth hormone in blood or urine of a human subject; and a method of confirming external administration of a human growth hormone.

More particularly, the above methods may comprise the steps of:

(a) measuring each concentration of 2 types of human growth hormone in blood or urine of a human subject to which at least one of the human growth hormones is externally administered,
(b) calculating their ratio and
(c) determining the concentration of the externally-administered human growth hormone(s) or confirming the external administration.

These methods may also comprise the steps of:

(i) measuring each concentration of two types of human growth hormone in blood or urine of a human subject at normal time to which no human growth hormone(s) has been administered,
(ii) calculating their total concentration measured in step (i) and a ratio of the concentration of one or each of the human growth hormones to the total concentration thus obtained,
(iii) measuring each concentration of the two types of human growth hormones in blood or urine of the human subject after administration of at least one of the human growth hormones or at the time when administration is suspected,
(iv) calculating their total concentration measured in step (iii) and a ratio of the concentration of one or each of the human growth hormones to the total concentration thus obtained, and
(v) determining the concentration of the externally-administered human growth hormone(s) or confirming the external administration by comparing the ratio(s) in step (ii) with the ratio(s) in step (iv).

It is known that there are two kinds of human growth hormones (abbreviated as hGH) which ore produced through alternative splicing on the same gene, one having a molecular weight of about 22,000 (abbreviated as 22K hGH) and the other having a molecular weight of about 20,000 (hereinafter abbreviated as 20K hGH). 22K hGH is a protein comprising 191 amino acids. It is also known that this 22K hGH accounts for about 90% of the total hGH in blood. On the other hand, 20K hGH is a protein comprising 176 amino acids. 20K hGH is present in blood in lesser amounts compared with 22K hGH and has been estimated to account for about 10% of the total hGH in-blood. Both 20K hGH and 22K hGH are proteins encoded originally in the same gene. For this reason, the structures of both proteins, i.e., their amino acid sequences are very similar to each other. The only difference between them is that 20K hGH has a structure deletion of 15 amino acid residues corresponding to the 32nd to 46th amino acid residues from the N-terminus of 22K hGH.

By utilizing recombinant DNA technology, 22K hGH has been produced abundantly for more than 10 years and an antibody specific to it has been obtained. This resulted in the development of an immunochemical assay specific to 22K hGH. On the other hand, it had been difficult to express 20K hGH by recombinant DNA technology and, therefore, it had been impossible to obtain a recombinant 20k hGH having the same structure as natural-type 20K hGH in a high purity as well as in large amounts. Therefore, a method of specifically measuring 20K hGH had not been provided.

However, by using a *Escherichia coli*, transformant, a method of obtaining such natural-type 20K hGH in a high purity and in large amounts has been developed recently (Uchida et al., J.Biotechnology:55:101-112(1997)). Moreover, a monoclonal antibody, which reacts specifically with the natural-type 20K hGH and does not substantially undergo a cross-reaction with 22K hGH, has been prepared and also a method of measuring a blood concentration of 20K hGH by using such an antibody specific to 20K hGH has been developed (EP Publication No.0814091).

Recently, since 22K hGH has been supplied in relatively large amounts, various physiological activities of 22K hGH have been further revealed. At first, hGH was used for the treatment of growth hormone deficient disease in children, however, the hGH therapy has been expansively indicated for such diseases as adult human growth hormone deficiency. Presently, the hGH therapy has been indicated further for dwarfism not caused by deficiency in secretion of hGH, and additionally for burn and/or wound, postoperative bad conditions, osteoporosis, etc. If hGH is used in clinical study for therapy of such diseases, it is necessary to grasp behaviors of hGH that has been externally administered. However, in the case of a patient whose hGH deficiency is incomplete, since the patient originally produces hGH which remains in blood, it is difficult only by measuring the blood hGH concentration to determine whether the blood hGH concentration value concerns the internally produced hGH in the patient (referred to as physiological hGH) or the hGH administered externally. No measurement method in such a case has been so far known.

On the other hand, while hGH has been used as medicines, it is also well known that hGH has been on subject to be tested for doping, as one of forbidden drugs in sports for recent years.

However, it is difficult to determine whether or not hGH has been administered to a human subject simply based on a high or low blood hGH concentration obtained by such measurement for the following reasons:

(1) The detection of hGH is difficult because its blood concentration is so low as several ng/ml to several tens ng/ml even in the case of 22K hGH which is estimated to be present in blood in larger quantities compared with 20K hGH.
(2) Even if the detection of hGH is possible, its blood concentration remarkably differs depending on individuals, sex and age.
(3) Since hGH is intermittently secreted from a pituitary gland, the blood hGH concentration of even the same subject differs depending on the time when blood is drown.

In addition, since secondary physiological reactions ,which ore induced by hGH, differ greatly depending on individuals, sex and age and further and since correlation between the secondary physiological reactions and externally-administered hGH is considered to be complex, the blood hGH concentration alone could not provide a proper index suitably appropriate for a doping test.

Presently, 22K hGH has been an subject to be tested for doping. However, 22K hGH is believed to account for the largest part of the total hGH in human body and, therefore, specific measurement of 22K hGH alone could not provide correct results of the doping tests.

20K hGH has been estimated to account for about 10% of the total hGH in blood based on respective measurement of blood concentrations of each of the total hGH and 22K hGH. However, since the method of measuring 20K hGH specifically has been developed very recently as described above, an exact existence ratio between 22K hGH and 20K hGH had not yet been measured. It is thus needless to say that there was no information of daily change of the existence ratio between them or change which is caused by:

(a) difference in individuals, sex and age, and
(b) a rise in the blood hGH concentration induced by accelerating the secretion of hGH in tolerance tests.

An subject matter of the present invention is a method of determining or judging whether or not hGH has been externally administered and a method of measuring a blood or urine hGH concentration that has been externally administered without including physiological hGH by using either a ratio between the 22K hGH and 20K hGH concentrations in blood or urine as an index; or ratios of one or each of the 22K hGH and 20K hGH concentrations to the total hGH concentration measured at normal time (i.e., at a time when any hGH has not been administered) and at a time after a hormone has been administered, respectively.

The present inventors measured blood concentrations of 22K hGH and 20K hGH with a measurement method specific for 22K hGH and 20K hGH, respectively, using various kinds of human blood plasmas and sera, and then calculated the ratio of concentrations of them. Also, an investigation was made on how the ratios between blood concentrations of 22K hGH and 20K hGH varied depending various factors. The results have shown that the concentration of each of 22K hGH and 20K hGH varies greatly depending on individuals, sex and age, and its daily variation is also large. It was also found that the concentration was made remarkably high by tolerant tests in some cases. However, it has further found for the first time that a ratio of the concentration of 20K hGH (or 22K hGH) to that of the total hGH (the sum of concentrations of 22K hGH and 20K hGH) is almost same in different individuals, sex and age. To our surprise, it has also been found that, although the total hGH concentration varies greatly all day long, the above ratio remains almost constant and that, even if the concentration of the total hGH concentration in blood varies by tolerant tests, said ratio is not much influenced thereby.

These new findings and results have also revealed that, when either 22K hGH or 20K hGH is singly administered externally to a human subject, the ratio between blood concentrations of 22K hGH and of 20K hGH varies before and after the administration. This suggests that, by measuring the blood concentration of each of 22K hGH and 20K hGH and by monitoring their ratio, it becomes possible to determine or judge whether or not hGH has been externally administered and to calculate the blood hGH concentration that has been administered. Also, it is expected to be possible to draw the same conclusion even if a urine hGH concentration, instead of the blood hGH concentration, is used as an subject to be measured.

The present invention has been thus made on the basis of the above new knowledge of the present inventors.

That is, the present invention provides a method of determining hGH concentrations on the basis of a ratio between blood or urine concentrations of 22K hGH and 20K hGH. The present invention also provides a method of determining or confirming whether or not hGH have been externally administered to a human subject on the basis of a ratio between blood or urine concentrations of 22K hGH and 20K hGH.

The determination of the externally-administered hGH concentration or the confirmation of the external hGH administration according to the present invention can be also carried out by comparing a ratio of one or each of 22K hGH concentration and 20K hGH concentration to the total hGH concentration in blood or urine at normal time with that after administration of one of them or at the time when administration of one of them is suspected.

According to the present invention, a blood or urine hGH concentration that has resulted from external hGH administration can be determined. That is, the present invention enables easy and exact measurement of the blood or urine hGH concentration resulting from external administration.

The measurement method of the present invention is very useful for monitoring internal behaviors of externally-administered hGH in clinical applications of 20K hGH or 22K hGH. Also, the present invention enables the confirmation or judgement as to whether or not hGH has been administered externally. Accordingly, the method of the present invention is useful for doping tests in sports.

Embodiments of the present invention will now be described in more detail with reference to the accompanying drawings, in which:

FIG. 1 is a graph showing a standard curve in immunological measurement of 20K hGH.
FIG. 2 is a graph showing a standard curve in immunological measurement of 22K hGH.
FIG. 3 is a graph showing relation between secretion of 20K hGH and age.
FIG. 4 is a graph showing relation between secretion of 22K hGH and age.
FIG. 5 is a graph showing relation between a physiological ratio of 20K hGH and age.
FIG. 6 is a graph showing correlation between serum 20K hGH and 22K hGH levels in normal human subjects.
FIG. 7 is a graph showing relation between a physiological ratio of 20K hGH and height.
FIG. 8 is a graph showing relation between physiological ratio of 20K hGH and weight.
FIG. 9 is a graph showing relation between a physiological ratio of 20K hGH and body fat.
FIG. 10 is a graph showing behaviors of hGH in blood observed while a children is sleeping.
FIG. 11 is a graph showing behaviors of hGH in blood observed for about one month in an adult.
FIG. 12 is a graph showing behaviors of hGH in blood which has been increased by insulin tolerance tests.
FIG. 13 is a graph showing behaviors of hGH in blood which has been increased by clonidine tolerance tests.

The present invention will be hereinafter described in detail.

Human growth hormones to be measured according to the present invention is a general term for hormones including 22K hGH and 20K hGH. 20K hGH is the one being present in human blood or urine, which is a protein comprising 176 amino acids. Two kinds of 20K hGH is known, one has methionine at the fourteenth position from the N-terminus and the other has serine at the corresponding position, and either of them may be used in the present invention. 20K hGH to be handled in the present invention can be obtained according to a genetic recombination method disclosed in, for example, EP Publication No.0587427. 22K hGH to be handled herein is the one being present in human blood or urine, which is a protein consisting of 191 amino acids. 22K hGH is commercially available, for example, under a brand name "Genetropin" (Pharmacia Upjohn). To measure concentrations of 22K hGH and 20K hGH in human blood or urine, immunoassaies using antibodies may be used. High sensitive measurement is possible, in particular, if a monoclonal antibody is used as an antibody for detection. In the case of the immunoassay using an antibody, the measurement sensitivity is preferably 10 pg/ml or less than 10 pg/ml.

Also, in addition to the above immunoassay, electrophoresis such as SDS electrophoresis, various types of column chromatography, high-performance liquid chromatography (HPLC) and the like may be used for the measurement of concentrations of 22K hGH and 20K hGH in blood or urine, and the combined methods of such chromatography method with the immunoassay may be possible as well. To measure concentrations of 22K hGH in human blood, an immunochemical measurement method using a monoclonal antibody reacting specifically with 22K hGH, as disclosed in Japanese Patent Laid-Open (Kokai) No. 273496/'88, can be used. The monoclonal antibody to be used herein can be obtained from a hybridoma produced by cell fusion of anti-22K hGH antibody-producing cells with myeloma cell strains. A36020047P (BiosPacific) is one example of the monoclonal antibody of this kind.

On the other hand, to measure concentrations of 20K hGH in human blood, an immunochemical measurement method using a monoclonal antibody reacting specifically with 20K hGH and substantially not reacting with 22K hGH, as disclosed in EP Publication No.0814091, may be used. The monoclonal antibody to be used herein can be obtained from a hybridoma produced by cell fusion of anti-20K hGH antibody-producing cells with myeloma cell strains. MTC6A cell strain (FERM BP-5913) is one example of such a hybridoma. This MTC6A has been deposited at National Institute of Bioscience and Human-technology, Agency of Industrial Science and Technology of the Ministry of International Trade and Industry (Higashi 1-1-3 Tsukubacity Ibaragi Prefecture, Japan) with accession No. FERM BP-5913 under the Budapest Treaty.

To produce the desired monoclonal antibody reacting specifically with 20K hGH using the anti-monoclonal antibody-producing hybridoma, the hybridoma can be cultured according to a conventional method including a cell culture method or an ascitic fluid forming method. The monoclonal antibody thus produced is then purified from supernatant of the resulting culture or ascitic fluid. The purification of the monoclonal antibody from the culture supernatant or ascitic fluid can be performed by a conventional method. A method by appropriately combing, for example, ammonium sulfate fractionation, gel filtration, ion exchange chromatography, affinity chromatography and the like is available as well.

The method of immunological measurement of 22K hGH and 20K hGH using a monoclonal antibody refers to the method using the monoclonal antibody obtained by the methods described above. According to the present invention, the immunological measurement method to be used herein includes, for example, enzyme immunoassay, radioimmuno-assay, fluoroimmunoassay, luminescent immunoassay and the like, and the enzyme immunoassay out of them is preferably used. More preferably, a sandwich enzyme immunoassay using a monoclonal antibody binding insoluble carrier obtained by binding the said monoclonal antibody to an insoluble carrier is used.

The method for measuring 20K hGH based on the sandwich enzyme immunoassay is hereafter described. The method for measuring 22K hGH is the same as for 20K hGH except that 20K hGH should be replaced with 22K hGH.

As a first step, the monoclonal antibody is immobilized on an insoluble carrier and the immobilized monoclonal antibody is subjected to reaction with the test solution containing human growth hormones, whereby a binding product having 20K hGH alone specifically bound to the immobilized monoclonal antibody on the insoluble carrier is formed.

The insoluble carrier includes a microplate, plastic beads, glass beads, magnetic fine particles, etc. To bind the monoclonal antibody to such insoluble carriers, known chemical binding method is usable and a physical adsorption method will do for the purpose. That is, the monoclonal antibody used in the present invention is dissolved in a carbonic acid buffer, phosphate buffer, etc. and to the solution the insoluble carriers are added. After the resulting mixture is left at 0°C to room temperature for not less than one hour, it is washed with Tween 20 (polyoxyethylene sorbital monolaurylate), Tris hydrochloride buffer containing sodium azide and the like, phophate buffer, etc., to remove unbound antibodies. Then, the obtained insoluble carrier having the immobilized antibody can be used in the above first step.

As a second step, the bonding product on the insoluble carrier thus obtained in the first step is subjected to reaction with an enzyme-labeled anti-hGH antibody (hereinafter referred to as anti-hGH enzyme-labeled antibody), whereby a sandwich complex is formed on the insoluble carrier, which has a structure of anti-20K hGH monoclonal antibody-20K hGH-anti-hGH enzyme-labeled antibody.

As the anti-hGH antibody to be used as a material for the anti-hGH enzyme-labeled antibody, either the antibody which is reactive with only 20K hGH and recognizes an epitope different from that recognized by the anti-20K hGH monoclonal antibody; or the antibody which is reactive with both 20K hGH and 22K hGH can be used. These antibodies may be monoclonal or polyclonal. These antibodies can be obtained by a known method. Monoclonal antibody D14 is one of examples that can obtained by such a known method.

Enzymes with which anti-hGH is labeled include a horseradish peroxidase, alkaline phosphatase, glucose oxidase, β-galactosidase, etc.

Labeling an anti-hGH antibody or $F(ab')_2$ and Fab' fragments of the antibody with an enzyme can be achieved by known methods wherein sugar chains of the enzyme are oxidized by a periodic acid and the resulting aldehyde group is subjected to reaction with amino acids of the anti-hGH antibody; or wherein a maleimide group or pyridyl sulfide group is introduced into the enzyme and the group is subjected to reaction with thiol group in Fab' gragment of the anti-hGH antibody.

As a third step, the activity level of the enzyme used for labeling the sandwich complex obtained in the second step is measured. That is, since the activity level of the enzyme depends on the amount of 20K hGH allowed to react first, the measured level of the activity corresponds to the amount or concentration of 20K hGH alone contained in the test solution. The activity of the enzyme can be measured by adding substrate, enabling one to measure the enzyme activity.

Alternatively, the method may be also modified by using an anti-hGH biotin-labeled antibody, i.e., an anti-hGH antibody labeled with biotin, instead of the anti-hGH enzyme-labeled antibody. In this case, after a sandwich-like complex having a structure of anti-20K hGH monoclonal antibody-20K hGH-anti-hGH biotin-labeled antibody is formed on the insoluble carrier, the complex is subjected to reaction with enzyme-labeled avidin or enzyme-labeled streptoavidin, resulting in the formation of a sandwich-like complex having a structure of anti-20K hGH monoclonal antibody-20K hGH-anti-hGH biotin-labeled antibody-enzyme-labeled avidin or enyzme-labeled streptoavidin. After that, activity level of the enzyme bound to the sandwich-like complex is measured.

In addition, in the sandwich enzyme immunological measurement method described above, the anti-20K hGH monoclonal antibody (or the anti-22K hGH monoclonal antibody) may be alternatively used instead of the anti-hGH enzyme-labeled antibody as well.

The test samples to be measured by the immunological measurement method include human blood, human urine, other human body fluids, diluted solution thereof, their solutions purified or treated for pH adjustment, etc.

20K hGH and 22K hGH manufactured by a genetic recombination method can be used as a standard 20K hGH and 22K hGH for the creation of a calibration curve used in the immunological measurement.

The method of determining an externally administered hGH concentration in blood or urine by using blood or urine hGH concentrations measured by the method described above con include a step of calculating the externally administered hGH concentration on the basis of a ratio of blood or urine concentrations of 20K hGH and 22K hGH to those of the total hGH (the sum of blood concentrations of 22K hGH and 20K hGH; or the sum of urine concentrations of 22K hGH and 20K hGH).

The calculation on the basis of a ratio between blood or urine concentrations of 20K hGH and 22K hGH can be carried out as follows:

Firstly, concentrations of each of 22K hGH and 20K hGH in blood or urine taken from a patient at a time when obviously no human growth hormone has been administered are measured. The ratio between the blood or urine concentrations of the obtained 22K hGH (or 20K hGH) and those of the total hGH is determined and defined as a physiological ratio (for example, [20K hGH/(20K hGH + 22K hGH)]×100 ; or [22K hGH/(20K hGH + 22K hGH)]×100 .

This physiological ratio is almost stable in all humans and it exhibits a constant value which is not influenced by a time and/or amounts of the total hGH concentration. It is preferred that the measurement were carried out several times by changing the time and day to be measured, and averaging the values obtained in order to more exactly determine the physiological ratio.

Secondly, using the same method as above, the actual concentration of 20K hGH (or 22K hGH) is then measured in the comparative sample, which is taken from blood or urine of the same patient, whose the physiological ratio has been already measured, at a time after human growth hormones have obviously been administered or when the administration thereof has been suspected.

Based on the measured values, a ratio of a concentration of 22K hGH (or 20K hGH) to the total hGH concentration is calculated. Thin ratio based on the actually measured values to be compared is referred to as the measurement ratio.

Finally, on the basis of a difference obtained by comparing the physiological ratio and the measurement ratio obtained from the above steps, amounts of hGH externally administered are calculated.

If it is obvious that either of 22K hGH or 20K hGH has been administered, the hGH other than the administered hGH (e.g., 20K hGH if 22K ,hGH has been administered) is selected as a standard hGH. The concentration of the physiological hGH in the comparative sample can be determined based on the blood or urine concentration of the standard hGH in the comparative sample. The blood or urine concentration of hGH increased by the administration is determined from the difference between the concentration of the physiological hGH in the comparative sample and the actually measured hGH concentration in the comparative sample.

For example, if it is obvious that 22K hGH is administered, the physiological ratio concerning 20K hGH is used as a standard ratio. The ratio of a concentration of physiological 22K hGH to that of physiological 20K hGH or their total remains constant also when the measurement ratio is calculated using the comparative sample. This means that the concentration of physiological 22K hGH in the comparative sample can be determined using the actually measured 20K hGH concentration in the comparative sample and the physiological ratio between the 20K hGH and 22K hGH.

The difference value between the concentration of physiological 22K hGH in the comparative sample and the actually measured concentration of 22K hGH in the comparative sample correspond to the increased concentration of 22K hGH caused by its administration.

It is not always necessary to carry out the measurement of the physiological ratio prior to the determination of the measurement ratio, i.e., the measurement of the physiological ratio may be made after the completion of the determination of the measurement ratio, if the blood or urine hGH level of the human subject returns to the normal level.

The method of the present invention can be preferably applied to single administration of 20K hGH or 22K hGH. The method of the present invention can be also applied to their mixed administration, but when their mixed ratio corresponds to or is close to the physiological ratio, other determining methods may be used.

The method for measuring the urine concentration of the administered hGH using the concentration of 20K hGH and 22K hGH in urine includes a method wherein the administered hGH is calculated from ratios of concentrations of 20K hGH or 22K hGH to those of the total obtained hGH (the sum of urine concentrations of 22K hGH and of 20K hGH) obtained. Details of the measurement method are the same as in the case of using the blood as a test sample.

Methods of confirming for judging whether or not hGH has been administered will be hereinafter described.

According to the present invention, in order to confirm or judge whether or not hGH has been administered, as in the case of measuring administered hGH using the physiological ratio and the measurement ratio, after blood (or urine) concentrations of each of 20K hGH and 22K hGH are measured, the physiological ratio and measurement ratio are to be determined separately.

In the method to confirm or judge whether or not hGH has been administered, unlike in the case of measuring the concentration of the administered hGH, the only thing to be done is to confirm that a physiological ratio has been different from the measurement ratio. For example, if the measurement ratio concerning 22K hGH has become larger than its physiological ratio, one can judge that 22K hGH has been administered, while, if it has become smaller than 22K hGH, one can judge that 20K hGH has been administered.

The present invention comprehends a measuring kit to be used for measuring blood or urine concentrations of hGH administered or a judging kit to be used for judging whether or not hGH has been externally administered.

These kits comprise a reagent, antibody, solvent, washes, medical appliance, etc., required to immunologically measure blood or urine concentrations of 22K hGH and 20K hGH which are intended to serve easier practice of measuring and judging methods of the present invention.

The present invention also comprehends a machine or equipment wherein the method for measuring the blood or urine concentration of hGH that has been administered or for judging whether or not hGH has been externally administered is automated. In these machines or equipment, procedures have been automated to more easily execute the measuring method and judging method of the present invention. Equipment embedded with such automation programs is included within the scope of the present invention.

Example 1

Measurement of concentrations of 22K hGH and 20K hGH in human blood

(1) Immunological measuring method of 20K hGH

(1)-1 Preparation of monoclonal antibody

MTC6 as a monoclonal antibody specific to 20K hGH was isolated from the hybridoma MTC6A strain (FERM BP-5913) according to the method disclosed in EP Publication No.0814091. A36020047P as a monoclonal antibody specific for 22K hGH was purchased from BiosPacific. D14 as a monoclonal antibody which is not competitive with both MTC6A and A36020047P and able to react with both 20K hGH and 22K hGH was prepared according to the method for preparation of the above MTC6A.

Dissociation constants of 20K hGH and 22K hGH for three kinds of monoclonal antibodies, MTC6A, A36020047P and D14 were obtained by using BIAcore (Pharmacia) and the results are shown in Table 1. The symbol "ND" in Table 1 represents that no binding property was found in the reaction using BIAcore.

Table 1

| Dissociation constant of anti-hGH monoclonal antibodies | | |
|---|---|---|
| Monoclonal antibody | Dissociation constant | |
| | 20K hGH | 22K hGH |
| MTC6A | $4.4 \times 10^{-10}$ | ND |
| A36020047P | ND | $2.8 \times 10^{-10}$ |
| D14 | $4.6 \times 10^{-10}$ | $5.7 \times 10^{-10}$ |

(1)-2 Preparation of enzyme-labeled antibody

Enzyme labeling of antibodies was carried out according to methods reported by Ishikawa, et al (Nonisotopic Immunoassay; Ngo, T.T; Plenum Press; P27-55 [1988]).

SH group was introduced into the purified monoclonal antibody D14 by using S-acetylmercaptosuccinic anhydride and the resulting monoclonal antibody was allowed to react with maleimidized horseradish peroxidase (hereinafter POD) and POD-labeled D14 was obtained.

(1)-3 Measurement of blood concentration of 20K hGH

The blood concentration of 20K hGH was measured as follows.

The monoclonal antibody MTC6A which binds specifically to 20K hGH was diluted with PBS to give a concentration of 40µg/ml and 60µl of the diluted solution was dispensed to each well of a 96-well microplate (Greiner) and was left stand two night at 4°C. This plate was flushed with a washing agent (10mM Tris hydrochloride buffer containing 0.05% Tween 20, pH8.0) and then blocked with Blockace (Dainippon Pharmaceutical). After the removal of Blockace, 100µl of reaction buffer (PBS containing 1% BSA, 10µg/ml Heterophilic Blocking Reagent [Scantibodies] and 1M NaCl) and 25µl of the standard 20K hGH or sample solution to be tested were added and shaken for 2 hours. After the plate was flushed with the washing agent, 100µl of the POP-labeled anti-hGH monoclonal antibody D14 (with a concentration of 0.5µg/ml) diluted and adjusted with phosphate buffer containing 0.5% BSA, 0.15M NaCl, 0.05% casein, 1% rabbit serum and 0.02% 8-anilino-1-naphthalene sulfonic acid ammonium salt (pH6.5) (hereinafter referred to as labeled antibody dilute solution) were added and shaken for 2 hours. After the plate was flushed with a washing agent, 100μl of POD substrate solution (65μg/ml) prepared by diluting 3,3',5,5'-tetramethylbenzidine with 100mM citrate buffer(pH3.8) was added and left stand for 30 minutes. 100 ml of 1N sulfuric acid was added to the reaction solution and the absorbance of the solution at 450 nm was measured with Immunoreader (Intermed) using the absorbance at 620 nm as control. Based on the resulting values the 20K hGH concentration was calculated using a calibration curve shown in Fig. 1. The rate of cross-reaction with 22K hGH was not more than 0.1%.

(2) Immunological measurement method of 22K hGH

In the method of measuring the blood concentration of 22K hGH, as a monoclonal antibody specific for 22K hGH, A36020047P (BiosPacific) out of commercially available hGH monoclonal antibody was selected. 22K hGH-specific antibody A36020047P was diluted with PBS to give a concentration of 10μg/ml and 60μl of the diluted solution was dispensed to each well of a 96-well microplate and was left stand two night at 4°C. After this plate was flushed with the washing agent, blocking was made on the plate using Blockace. After the removal of Blockace, 100μl of the reaction buffer solution and either the standard 22K hGH or 25 μl of the test solution were added and shaken for 2 hours. After the plate was flushed with the washing agent, 100μl of the POD-labeled anti-hGH monoclonal antibody D14 diluted with the labeled antibody dilute solution to 0.05μg/ml and adjusted therewith was added and shaken for 2 hours. After the flushing with a washing agent, 100μl of the POD substrate solution was added and left for 30 minutes. 100 ml of 1N sulfuric acid was added to the reaction solution and the absorbance of the solution at 450 nm was determined with Immunoreader using the sbsorbance determined at 620 nm as control. Boned on the resulting values, the 22K hGH concentration was calculated using a calibration curve shown in Fig. 2. The rate of cross-reaction with 20K hGH was not more than 0.1%.

(3) Relation between hGH secretion and difference in sex

Blood was drawn from healthy 275 males and healthy 263 females and concentrations of 22K hGH and 20K hGH in their blood were determined according to method: (1) and (2) described above. Based on the measured values, the physiological ratio was calculated to determine the ratio of the values between males and females. The results are shown in Table 2. The values in the table represent those of mean ± standard deviation. As a result, the blood concentrations of 22K hGH and 20K hGH (shown in the left and center columns in Table 2) varied very widely from person to person and the standard deviation was approximately double the mean values. Also, differences in values were found between males and females and the concentrations of both 22K hGH and 20K hGH were higher by about 50% in females than in males. On the other hand, the values of the physiological ratio (in the right column in Table 2) remain constant compared with those of hGH concentrations and their standard deviations were one-third the mean values, with no difference in the ratio between males and females.

Table 2

| | Numbers of test samples measured | 22K hGH concentration (pg/ml) | 20K hGH concentration (pg/ml) | 20K hGH x 100/(22K hGH+20K hGH) |
|---|---|---|---|---|
| Healthy male | 275 | 1070±1890 | 82±189 | 6.33±2.61 |
| Healthy female | 263 | 1680±2120 | 127±162 | 6.31±2.14 |
| Ratio between male and female | | 0.64 | 0.65 | 1.00 |

(4) Relation between hGH secretion and age

Using prepared samples of sera drawn from 460 healthy subjects aged from 0 to 79, blood concentrations of 20K hGH and 22K hGH were measured according to methods described in (1) and (2) described above to obtain the physiological ratio of 20K hGH. The results are shown in Figs. 3 to 6. As shown in these Figures, 20K hGH and 22K hGH concentrations varied widely from person to person (Figs. 3 and 4) and high correlation was found between concentrations of both hGHs (r = 0. 947) (Fig. 6), the physiological ratio remains constant (about 6%) regardless of age (Fig. 5).

(5) Variation in concentrations from person to person

Using blood drawn several times from subjects (A to F), the blood concentrations of 20K hGH and 22K hGH were measured according to methods described in (1) and (2) above to compare the dispersion of blood concentrations of hGH and the physiological ratios of 20K hGH. As shown in Tables 3 and 4, the blood concentrations of hGH varies widely from person to person, however, the physiological ratios of 20K hGH remain almost constant.

Table 3

| Variation in concentrations of 22K hGH and 20K hGH from person to person and ratio of concentrations of 20K hGH to those of total hGH (22K hGH + 20K hGH) | | | | |
|---|---|---|---|---|
| Individual subject | Times of blood collection | 22K hGH concentration (pg/ml) | 20K hGH concentration (pg/ml) | 20K hGHx100/(2 2K hGH+20K hGH) |
| A | 1 | 140 | 8.7 | 5.7 |
| | 2 | 920 | 33.9 | 3.6 |
| | 3 | 19860 | 1061 | 5.1 |
| B | 1 | 1490 | 99.6 | 6.3 |
| | 2 | 2530 | 155.7 | 5.8 |
| | 3 | 12400 | 768.8 | 6.0 |
| C | 1 | 34200 | 3877 | 10.2 |
| | 2 | 150000 | 15983 | 9.6 |
| | 3 | 77500 | 10362 | 11.8 |
| D | 1 | 600 | 72.2 | 10.7 |
| | 2 | 2360 | 332 | 12.3 |
| | 3 | 1290 | 174 | 11.9 |
| E | 1 | 4670 | 327 | 7.4 |
| | 2 | 5450 | 471 | 8.0 |
| | 3 | 680 | 43.9 | 6.1 |
| | 4 | 4090 | 335 | 7.6 |
| | 5 | 2890 | 161 | 5.3 |
| | 6 | 2800 | 195 | 6.5 |
| | 7 | 7420 | 486 | 6.1 |
| | 8 | 3350 | 264 | 7.3 |
| | 9 | 4990 | 356 | 6.7 |
| F | 1 | 2990 | 217 | 6.8 |
| | 2 | 1930 | 140 | 6.8 |
| | 3 | 190 | 10.7 | 5.2 |
| | 4 | 440 | 23.6 | 5.1 |
| | 5 | 2220 | 150 | 6.3 |
| | 6 | 5720 | 441 | 7.2 |
| | 7 | 4980 | 377 | 7.0 |
| | 8 | 5640 | 301 | 5.1 |

(6) Correlation among hGH physiological ratio, height, weight and body fat

Using prepared sera samples collected from 70 healthy adults, the blood concentrations of 20K hGH and 22K hGH were measured according to methods described in (1) and (2) above to obtain physiological ratios of the 20K hGH concentration. No correlation among physiological ratios of both hGHs, height, weight and body fat was found as shown in Figs. 7 to 9.

(7) hGH behaviors in blood with time (1)

Using prepared sera samples collected from children every 20 minutes after sleep on set, concentrations of 20K hGH and 22hGH were measured according to methods described in (1) and (2) above to obtain physiological ratios of 20K hGH. As shown in Fig. 10, almost simultaneous secretion peaks were found in both hGHs when their secretion was accelerated during night and no remarkable change in the ratio of concentrations of both hGHs was observed.

(8) hGH behaviors in blood with time (2)

Using prepared sera samples collected from the same healthy adults as above every four days, concentrations of 20K hGH and 22K hGH were measured according to methods described in (1) and (2) above to obtain physiological ratios of 20K hGH. As shown in Fig. 11, no remarkable change in the ratio of the concentrations of both hGHs was noted during a period of about one month.

(9) Measurement of blood concentrations of hGH at time of stimulated secretion of hGH

Using prepared sera samples collected from the same children as above when their secretion of hGH was stimulated by insulin or clonidine, concentrations of 20K hGH and 22K hGH were measured according to methods described in (1) and (2) above to obtain the measurement ratio of 20K hGH. As shown in Figs. 12 and 13, almost simultaneous secretion peaks were found in both these two isoforms when their secretion was stimulated during both tolerance tests and no remarkable change in the ratio of the concentrations of both hGHs was noted.

Example 2

Measurement of hGH administered

A child was subcutaneously administered with 22K hGH (2.75 U)(1mg) and a serum sample was prepared. Concentrations of 20K hGH and 22K hGH in the serum sample were measured according to methods described in (1) and (2) in Example 1 above to calculate the physiological ratio and measurement ratio. As shown in Table 4, the ratio of 20K hGH being 4.8% before administration was reduced greatly down to 0.4% four hours after the administration.

Table 4

| Change in ratio of 22K hGH after administration of 22K hGH | | | | | | |
|---|---|---|---|---|---|---|
| Subject | Before administration | | | After administration | | |
| | 22K hGH(pg/ml) | 20K hGH(pg/ml) | Physiological ratio (%) | 22K hGH(pg/ml) | 20K hGH(pg/ml) | Measurement ratio (%) |
| A | 210 | 10.7 | 4.8 | 36200 | 141 | 0.4 |

**Claims**

1. A method of determining a concentration of a human growth hormone (hGH) that has been administered on the basis of a ratio between bodily fluid, e.g. blood, concentrations of a human growth hormone having a molecular weight of about 22,000 (22K hGH) and a human growth hormone having a molecular weight of about 20,000 (20K hGH).

2. A method as claimed in claim 1, which comprises the steps of :

   (1) measuring a concentration of each of said human growth hormone having a molecular weight of about 22,000 (22K hGH) and said human growth hormone having a molecular weight of about 20,000 (20K hGH) at a time when human growth hormones have not been administered to a subject and then determining a ratio defined as a physiological ratio of the 20K hGH or 22K hGH concentration to the total hGH concentration (the sum of the 22K hGH and 20K hGH concentrations) obtained by said measurement,

(2) drawing blood from said subject at a time after human growth hormones have been administered or when the administration thereof has been suspected and then determining a ratio defined as a measurement ratio of the 20K hGH or 22K hGH concentration to the total hGH concentrations in the same manner as in said stop (1), and

(3) determining blood concentrations of human growth hormones that have been administered on the basis of a difference between said physiological ratio determined by said step (1) and said measurement ratio determined by said step (2).

**3.** A method as claimed in claim 2, wherein said measurement of blood concentrations of each of said human growth hormone having a molecular weight of about 22,000 and said human growth hormone having a molecular weight of about 20,000 is carried out by using antibodies, e.g. monoclonal, specific for each of said growth hormones.

**4.** A method of confirming or judging whether or not a human growth hormone (hGH) has been administered on the basis of a ratio between blood concentrations of a human growth hormone having a molecular weight of about 22,000 (22K hGH) and a human growth hormone having a molecular weight of about 20,000 (20K hGH).

**5.** A method as claimed in claim 4, which comprises the steps of :

(1) measuring a concentration of each of said human growth hormone having a molecular weight of about 22,000 (22K hGH) and said human growth hormone having a molecular weight of about 20,000 (20K hGH) at a time when human growth hormones have not been administered to a subject and then determining a ratio defined as a physiological ratio of the 20K hGH or 22K hGH concentration to the total hGH concentrations (the sum of the 22K hGH and 20K hGH concentrations) obtained by said measurement,

(2) drawing blood from said subject at a time after human growth hormones have been administered or when the administration thereof has been suspected and then determining a ratio defined as a measurement ratio of the 20K hGH or 22K hGH concentration to the total hGH concentrations in the same manner as in said step (1), and

(3) determining blood concentrations of human growth hormones that have been administered by comparing said physiological ratio determined by said step (1) with said measurement ratio determined by said step (2).

**6.** A method as claimed in claim 5, wherein said measurement of blood concentrations of each of said human growth hormone having a molecular weight of about 22,000 and said human growth hormone having a molecular weight of about 20,000 is made by using antibodies, e.g. monoclonal, specific for each of said growth hormones.

**7.** A method as claimed in claim 2, 3, 5 or 6, wherein said growth hormone that has been administered is a human growth hormone having a molecular weight of about 22,000, or a human growth hormone having a molecular weight of about 20,000.

**8.** A method of determining a urine concentration of a human growth hormone that has been administered on the basis of a ratio between urine concentrations of a human growth hormone having a molecular weight of about 22,000 (22K hGH) and a human growth hormone having a molecular weight of about (20K hGH).

**9.** A method of confirming or judging whether or not a human growth hormone (hGH) has been administered on the basis of a ratio between urine concentrations of a human growth hormone having a molecular weight of about 22,000 (22K hGH) and a human growth hormone having a molecular weight of about 20,000 (20K hGH).

**10.** A kit of components for performing the method claimed in any of claims 1 to 9, and equipment for performing said method, as described herein.

Fig.1

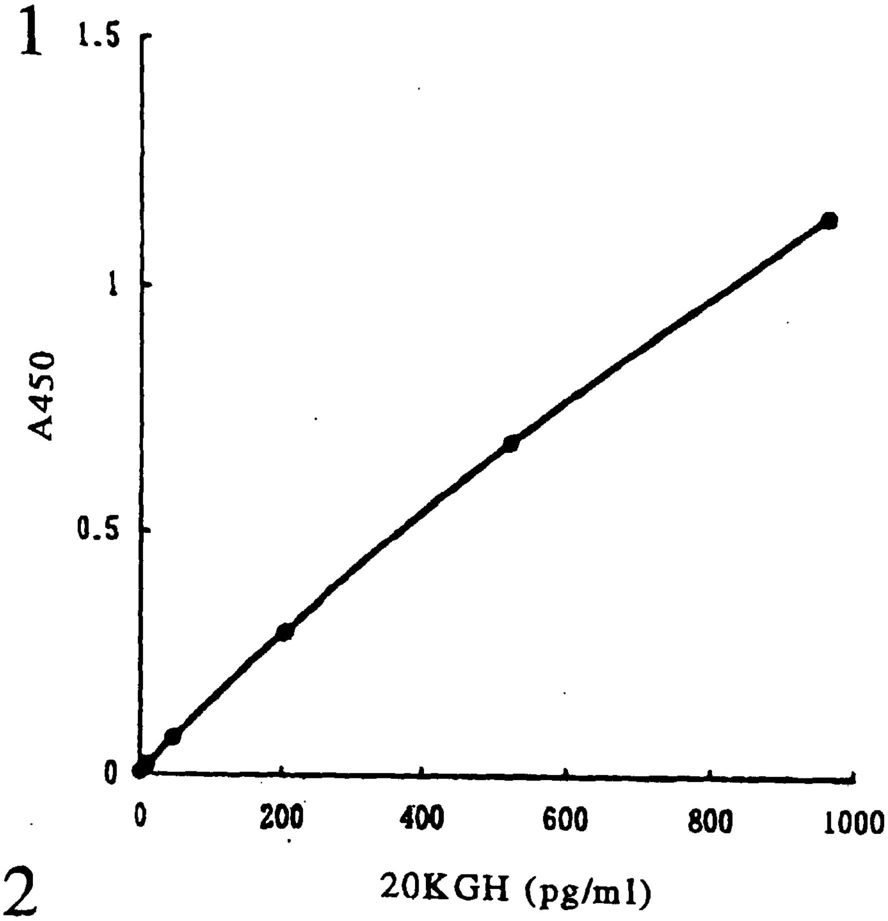
1.5
1
0.5
0
A450
0
200
400
600
800
1000
20KGH (pg/ml)

Fig.2

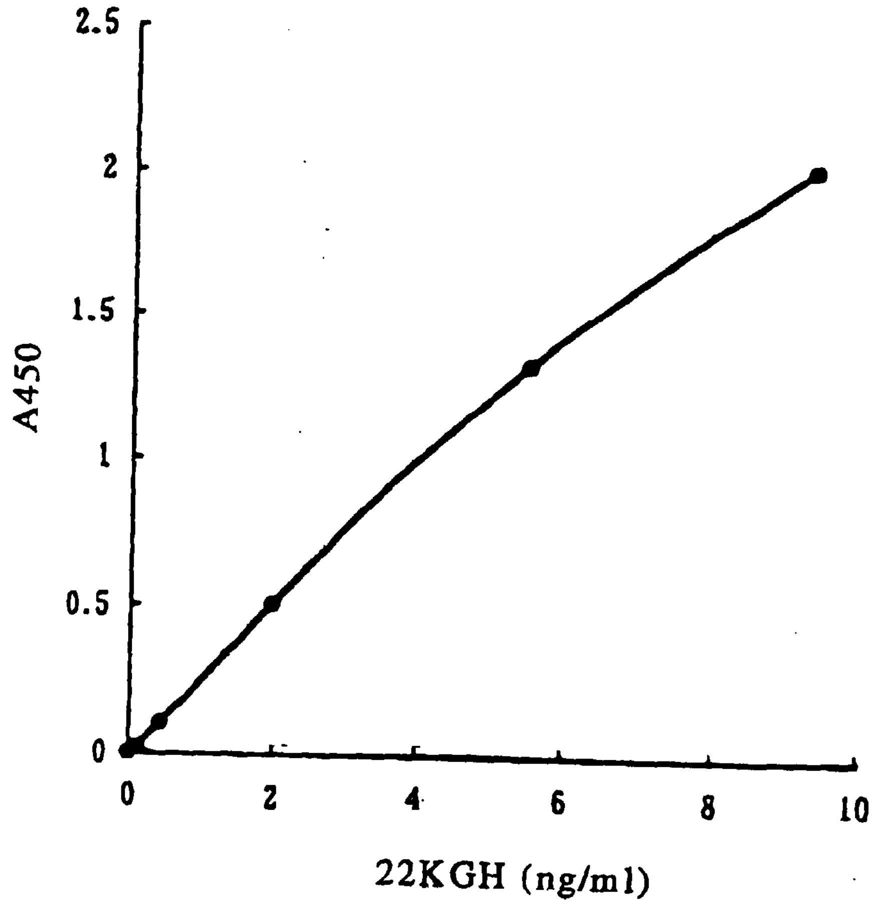
2.5
2
1.5
1
0.5
0
A450
0
2
4
6
8
10
22KGH (ng/ml)

Fig.3

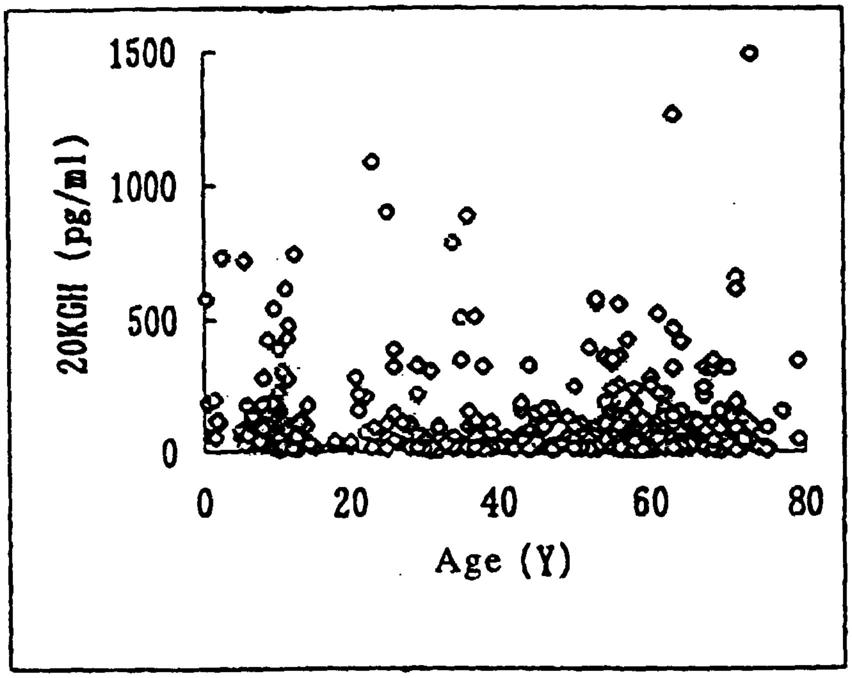
1500
1000
500
0
20KGH (pg/ml)
0
20
40
60
80
Age (Y)

Fig.4

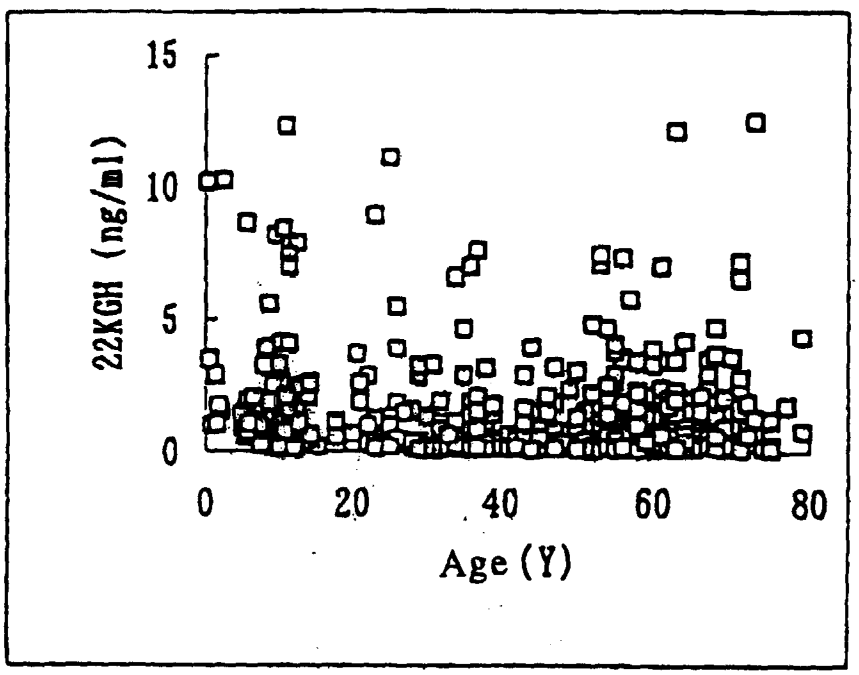
15
10
5
0
22KGH (ng/ml)
0
20
40
60
80
Age (Y)

Fig.5

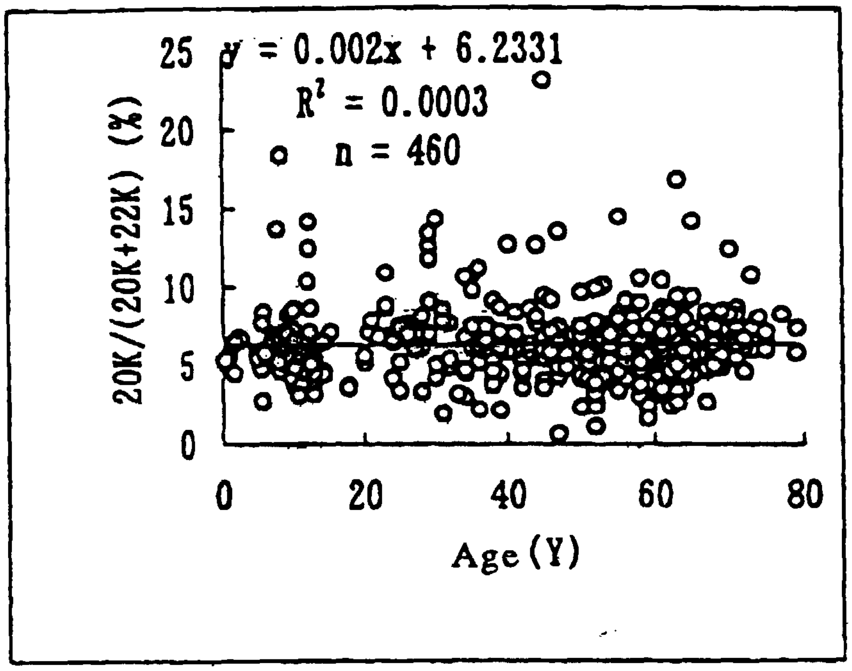
y = 0.002x + 6.2331
R² = 0.0003
n = 460
20K/(20K+22K) (%)
25
20
15
10
5
0
0
20
40
60
80
Age (Y)

Fig.6

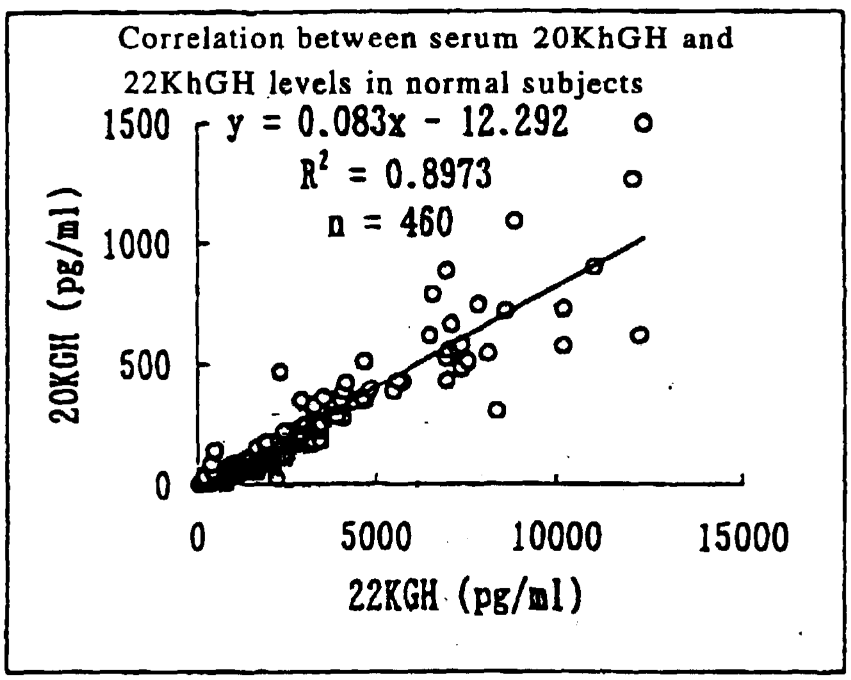
Correlation between serum 20KhGH and
22KhGH levels in normal subjects
y = 0.083x - 12.292
R² = 0.8973
n = 460
20KGH (pg/ml)
1500
1000
500
0
0
5000
10000
15000
22KGH (pg/ml)

Fig. 7

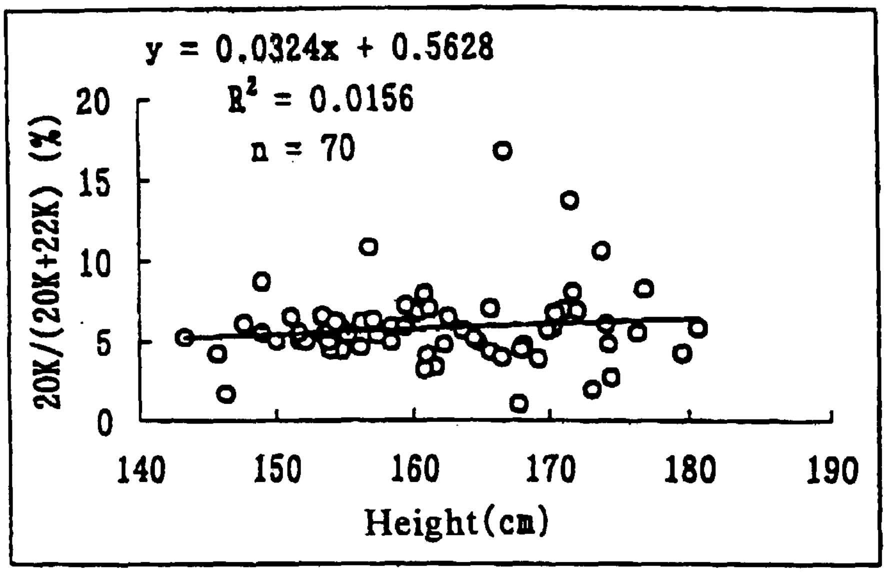


Fig. 8

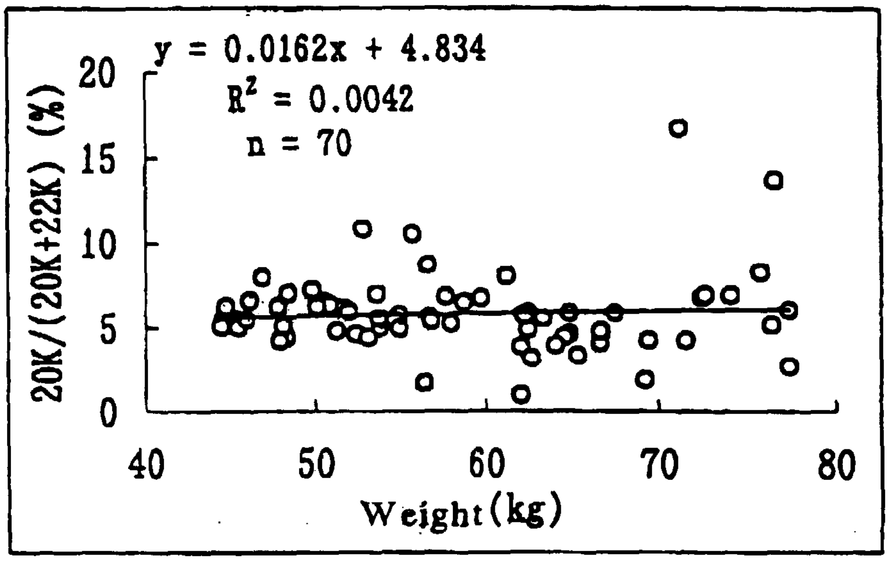


Fig. 9

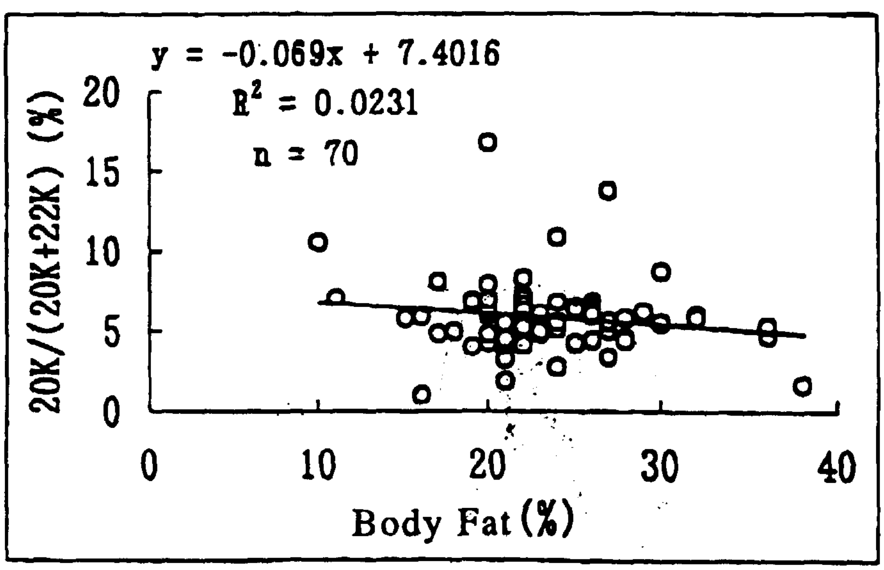

# Fig. 10

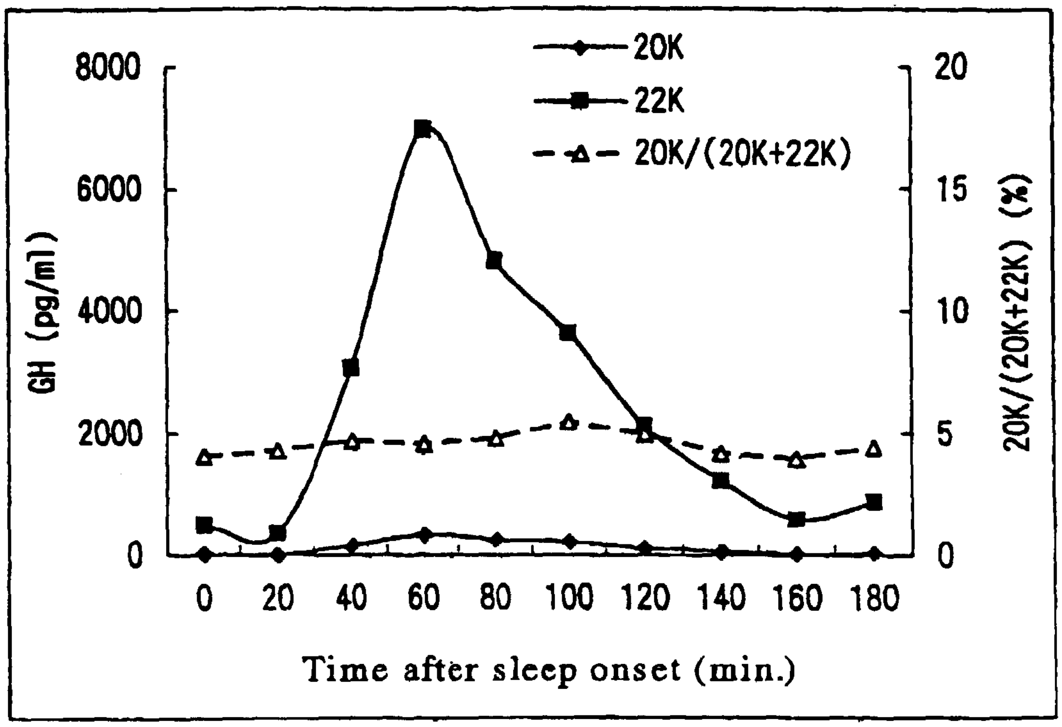
8000
6000
4000
2000
0
GH (pg/ml)
20K
22K
20K/(20K+22K)
20
15
10
5
0
20K/(20K+22K) (%)
0 20 40 60 80 100 120 140 160 180
Time after sleep onset (min.)

# Fig. 11

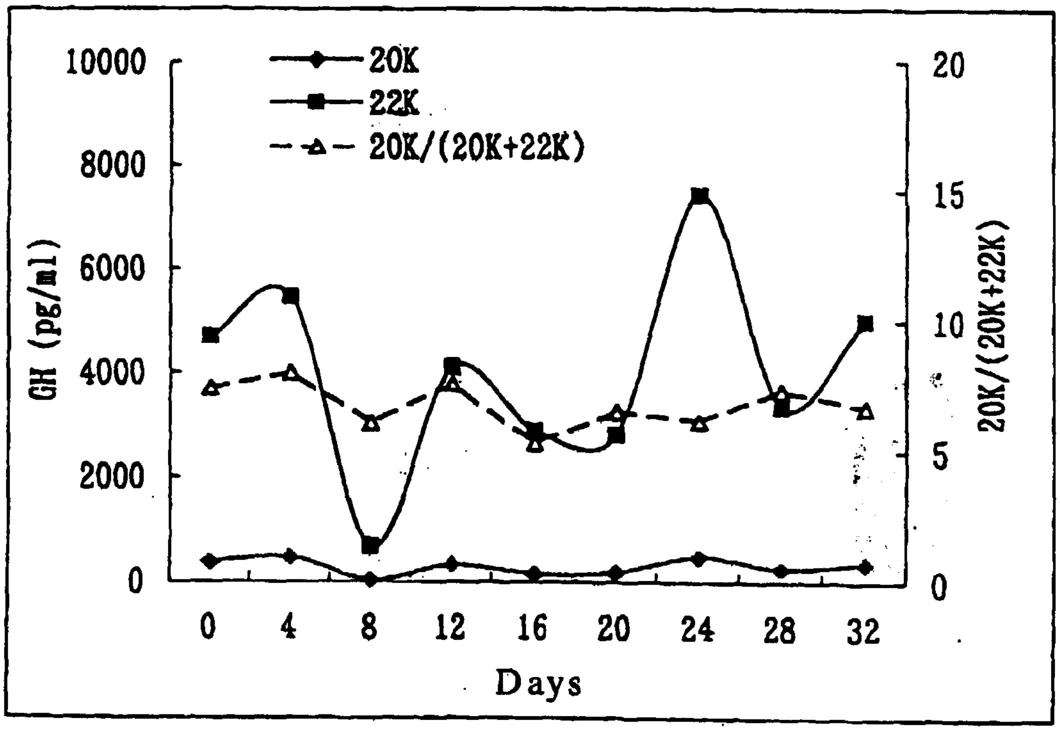
10000
8000
6000
4000
2000
0
GH (pg/ml)
20K
22K
20K/(20K+22K)
20
15
10
5
0
20K/(20K+22K)
0 4 8 12 16 20 24 28 32
Days

Fig.12

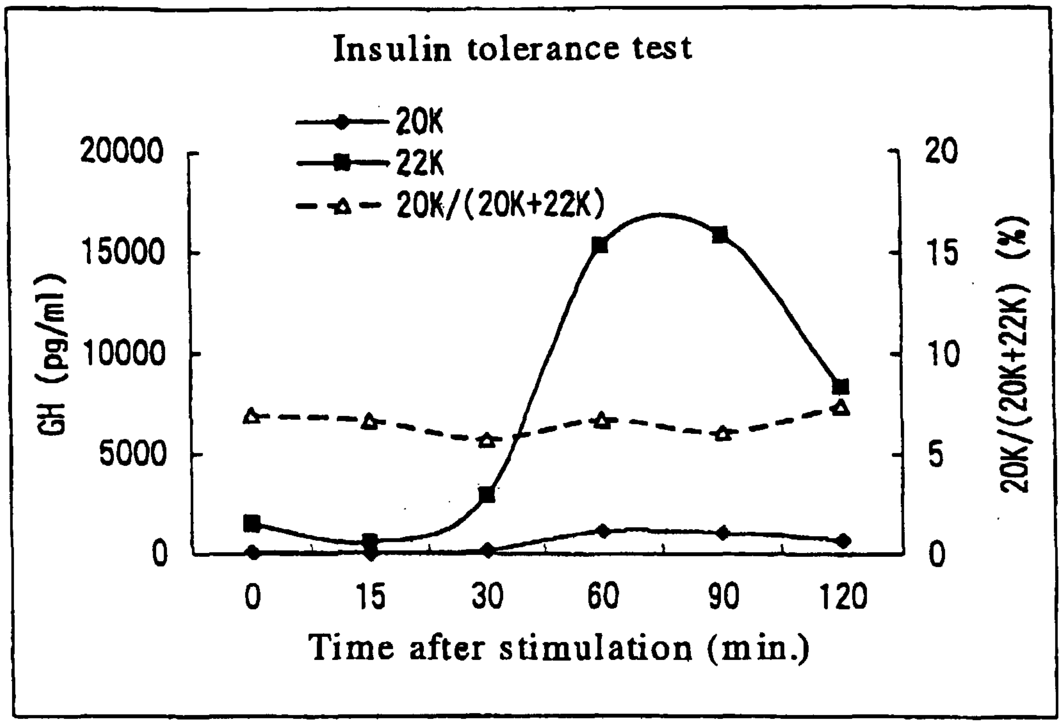
Insulin tolerance test
20K
22K
20K/(20K+22K)
20000
15000
10000
5000
0
GH (pg/ml)
20
15
10
5
0
20K/(20K+22K) (%)
0
15
30
60
90
120
Time after stimulation (min.)

Fig.13

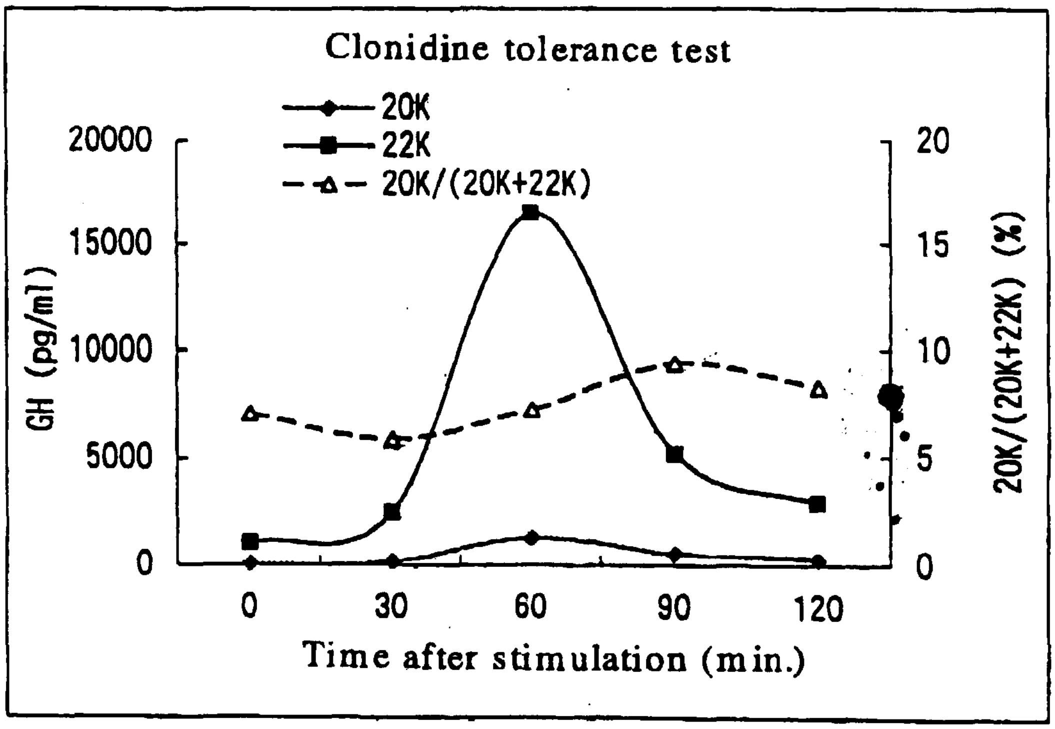
Clonidine tolerance test
20K
22K
20K/(20K+22K)
20000
15000
10000
5000
0
GH (pg/ml)
20
15
10
5
0
20K/(20K+22K) (%)
0
30
60
90
120
Time after stimulation (min.)

European Patent Office

# EUROPEAN SEARCH REPORT

Application Number
EP 99 30 2377

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X | BANFI, G. ET AL.: "Growth Hormone and Insulin-like Growth Factor I in Athletes Performing a Marathon at 4000m of Altidude" GROWTH REGULATION, vol. 4, no. 2, June 1994 (1994-06), pages 82-86, XP002108699 * abstract * --- | 1-10 | G01N33/577 G01N33/74 |
| X | FOSTER, CAROL M. ET AL.: "Bioactivity of Human Growth Hormone in Serum: Validation of an In Vitro Bioassay" ENDOCRINOLOGY, vol. 132, no. 5, 1993, pages 2073-2082, XP002108700 * abstract * * page 2078, column 1 - column 2, line 9 * * page 2080, column 2, paragraph 4 - page 2081, column 1, paragraph 2 * --- | 1-10 | |
| D,A | EP 0 814 091 A (MITSUI TOATSU CHEMICALS) 29 December 1997 (1997-12-29) * the whole document * --- | 1 | TECHNICAL FIELDS SEARCHED (Int.Cl.6) G01N |
| D,A | PATENT ABSTRACTS OF JAPAN vol. 013, no. 095 (C-573), 6 March 1989 (1989-03-06) & JP 63 273496 A (SUMITOMO PHARMACEUT CO LTD;OTHERS: 01), 10 November 1988 (1988-11-10) * abstract * ----- | 1 | |

The present search report has been drawn up for all claims

| Place of search | Date of completion of the search | Examiner |
|---|---|---|
| THE HAGUE | 9 July 1999 | Gundlach, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 99 30 2377

This annex lists the patent family members relating to the patent documents cited in the above-mentioned European search report.
The members are as contained in the European Patent Office EDP file on
The European Patent Office is in no way liable for these particulars which are merely given for the purpose of information.

09-07-1999

| Patent document cited in search report | Publication date | Patent family member(s) | Publication date |
|---|---|---|---|
| EP 0814091 A | 29-12-1997 | AU 690273 B | 23-04-1998 |
| | | AU 2491697 A | 22-01-1998 |
| | | JP 10072500 A | 17-03-1998 |
| | | NZ 328115 A | 26-02-1998 |
| JP 63273496 A | 10-11-1988 | NONE | |

EPO FORM P0459

For more details about this annex : see Official Journal of the European Patent Office, No. 12/82